# EUROPEAN PATENT SPECIFICATION

(11) **EP 0 639 083 B1**
(45) Date of publication and mention of the grant of the patent: **18.07.2001**
(21) Application number: 92915130.6
(22) Date of filing: 07.05.1992
(51) Int. Cl.: A61K 47/48, A61K 51/10

(54) **COMPLEXING AGENTS AND TARGETING IMMUNOREAGENTS**
KOMPLEXBILDNERMITTEL UND ZIELIMMUNOREAGENZIEN
COMPLEXANTS ET IMMUNOREACTIFS DE CIBLAGE

(43) Date of publication of application: 22.02.1995
(73) Proprietor: NYCOMED IMAGING AS, 0401 Oslo (NO)
(72) Inventor: TONER, John, L., Downingtown, PA 19335 (US); HILBORN, David, A., Henrietta, NY 14467 (US); MURRAY, Bruce, J., Walworth, NY 14568 (US); HOUSSAIN, Timothy, Z., Ithica, NY 14850 (US); SNOW, Robert, A., West Chester, PA 19380 (US); SAHA, Ashis, K., Frazer, PA 19355 (US); PHILION, Richard, Averill Park, NY 12018 (US); SHEARMAN, Clyde, W., West Chester, PA 19382 (US); SHAH, Chandra West Gate Village, 500, Malvern, PA 19355 (US)
(74) Representative: Canning, Lewis R.
(86) International application number: US9203858
(87) International publication number: WO9321957

(56) References cited:
- EP-A- 0 171 978
- EP-A- 0 178 450
- EP-A- 0 264 333
- EP-A- 0 288 256
- WO-A-90/00550
- WO-A-91/10645
- WO-A-92/08494
- WO-A-94/12216
- WO-A-94/20523
- VELI-MATTI MUKKALA ET AL.: "DEVELOPMENT OF LUMINESCENT EUROPIUM(III) AND TERBIUM(III) CHELATES OF 2,2':6',2''-TERPYRIDINE DERIVATES FOR PROTEIN LABELLING." HELVETICA CHIMICA ACTA, vol. 76, 1993, pages 1361-1378, XP002082606 BASEL CH
- VELI-MUKKALA ET AL.: "NEW HETEROARAOMATIC COMPLEXING AGENTS AND LUMINESCENCE OF THEIR EUROPIUM(III) AND TERBIUM(III) CHELATES." HELVETICA CHIMICA ACTA, vol. 75, 1992, pages 1621-1632, XP002082607 BASEL CH
- J. Heterocyclic Chem., vol. 18, May 1981, C.J. CHANDLER et al. "Synthesis of some 2,9-disubstituted-1,10-phenanthrolines", pages 599-601

## Description

### FIELD OF THE INVENTION

The present invention relates to novel immunoreagents and more particularly to targeting radioactive immunoreagents which find particular utility in therapeutic and diagnostic imaging compositions and methods. The present invention further relates to novel complexing agents.

### BACKGROUND OF THE INVENTION

Prior to 1980, the targeting of tumor-bearing sites by radioimmunoglobulin had been demonstrated by a number of laboratories at different institutions (S.E. Order et al., "Use of Isotopic Immunoglobulin in Therapy," Cancer Research 40, 3001-7 (August 1980)). By 1980 it was demonstrated that tumors would concentrate radiolabeled-antibodies to tumor associated antigens and that radiolabeled reagents employed allowed both diagnostic imaging of tumors, e.g., by gamma camera imaging (radioimmunoscintigraphy) and positron tomography, and therapeutic treatment, i.e., reduction in tumor size by the targeting radioactive immunoreagent.

Early targeting with radiolabeled immunoreagents was carried out with radioactive iodine. However, as noted by Scheinberg et al, "Tumor Imaging with Radioactive Metal Chelates Conjugated to Monoclonal Antibodies," Science 215, No. 19, 1511-13 (March 1982), iodine isotopes pose several problems, particularly with respect to scanning of tumor images. Of the three commonly available isotopic forms, only ¹²³I has the appropriate emission characteristics for imaging and a short enough half-life to be safely used diagnostically. The gamma radiation of ¹²⁵I is too weak for imaging. ¹³¹I has often been used but is undesirable because of its long half-life and high energy gamma and cytotoxic beta radiations. ¹³¹I has also been used therapeutically for large tumors, but appears ineffective in the treatment of small tumors. Moreover, rapid metabolism of radioiodinated antibodies allows incorporation of the iodine into the thyroid and active excretion of the iodine by the stomach and urinary tract. This dispersion of the radioactive iodine hinders imaging of specific tumors, since the tumors are hidden by background radiation.

In addition to tumor targeting with radioactive antibodies for diagnostic imaging and therapeutic treatment, similar targeting has been accomplished for diagnostic imaging of infarcts, specifically, myocardial infarcts, using antibodies to canine cardiac myosin [Khaw et al, "Myocardial Infarct Imaging of Antibodies to Canine Cardiac Myosin Indium - 111 - Diethylenetriamine Pentaacetic Acid," Science 209, 295-7 (July 1980)], and for imaging atherosclerosis by targeting atherosclerotic plaques. The same disadvantages in the use of radioactive iodine exist for diagnostic infarct imaging as for tumor imaging and therapeutic treatment.

It is known that ¹¹¹In can be complexed with polyaminocarboxylic acids such as ethylene diaminetetraacetic acid (EDTA) and diethylenetriaminepentaacetic acid (DTPA). However, the covalent linkage of proteins (antibodies) to these complexing agents, accomplished by acylation with activated carbonyls, aromatic diazonium coupling, or bromoacetylation, is inefficient, even though the isocyanatobenzyl derivatives described by Brechbiel et al ["Synthesis of 1-(p-Isothiocyanatobenzyl) Derivatives of DTPA and EDTA. Antibody Labeling and Tumor Imaging Studies," Inorg. Chem. 25, 2772-81 (1986)] were created to facilitate covalent attachment of proteins with the complexing agents.

Recently, research efforts have been directed to improved antibodies (Ab's), e.g., monoclonal, specific antibodies for specific targeting, antibodies that complex or bind directly with radionuclides, preferred radionuclides and combinations thereof with antibodies and complexing agents. Some attempts have been made towards improving complexing agents.

Nonetheless, EDTA and especially DTPA and derivatives thereof have remained the prevalent complexing agents to covalently bind antibody and coordinately complex metallic radionuclides. However, the inadequacies of DTPA have been noted, for example, by Parker et al, "Implementation of Macrocycle Conjugated Antibodies for Tumor Targeting," Pure and Appl, Chem., 61, No. 9, 1637-41 (1989): "Conventionally the metal radionuclide has been complexed by an acyclic chelate (e.g. EDTA or DTPA) which is covalently linked to the antibody. None of the chelates is adequate because the metal tends to dissociate in vivo,...", and by Cox et al, "Synthesis of a Kinetically Stable Yttrium-90 Labelled Macrocycle-Antibody Conjugate," J. Chem. Soc., Chem, Commun, 797-8 (1989): "Yttrium-90 is an attractive isotope for therapy...but its clinical use will be very limited because of bone marrow toxicity, resulting from acid-promoted release of ⁹⁰Y from an antibody linked chelate such as diethylenetriaminepentaacetic acid (DTPA)."

Previous attempts to develop improved complexing agents have provided materials which have their own shortcomings. For example, Craig et al "Towards Tumor Imaging with Indium-111 Labelled Macrocycle-Antibody Conjugates," J. Chem, Soc, Chem, Commun., 794-6 (1989) describe macrocyclic hexacoordinating ligands but state that "The limiting feature of this approach is that ¹¹¹In labelling of the macrocycle is required before antibody conjugation. Indium binding by (4) is insufficiently fast at 37°C for efficient radiolabeling.... Other tribasic triazamacrocyclic ligands were screened therefore for their ability to bind indium rapidly under mild conditions (20°C, pH 5, < lh), yet still form a kinetically stable complex in vivo.... However, only (6) proved effective when the ligand concentration was 10µM, and under these conditions a 96% radiolabeling yield was determined (30 min, pH 5, 20°C)."

Nevertheless, thirty minutes is still unsatisfactory. It would be highly desirable to have complexing agents superior to EDTA and DTPA which would coordinately bind preferred radionuclides such as In, Y, Sc, Ga, Ge, etc. within a few minutes, i.e., in less than about 5 minutes immediately prior to administration of the reagent to the patient, especially when a short-lived radionuclide must necessarily be generated from a longer-lived radionuclide at the time of treatment of the patient.

It should be noted that complexes of yttrium, a preferred radionuclide for therapy, tend to be less stable than those of indium [(Mather et al, "Labelling Monoclonal Antibodies with Yttrium 90," Eur, J. Nucl. Med. 15, 307-312 (1989)] with respect to conventional complexes. Mather et al teach that biodistribution studies in cancer patients using radiolabeled antibodies have suggested that the in vivo stability of yttrium-labeled antibodies is not as great as that of their ¹¹¹In-labelled counterparts and that these findings are supported by other recent publications in the field.

When chelating agents are covalently bonded to proteins (such as Ab's), the proteins usually are capable of accepting far more than one molecule of the chelating agent because they contain a host of amine and sulfhydryl groups through which the chelating agents are bound. It is often very important to determine how many chelating sites are bound to each protein molecule. The most convenient way to accomplish this is by spectrophotometric means. However, prior art chelating agents and chelates thereof have spectral absorptions that overlap with those of useful proteins, and an analytical determination of the number of chelating or chelated sites per molecule of protein cannot be made unequivocally by spectrophotometry since the overlapping spectral absorptions mask each other. It would thus be highly desirable to obtain chelating agents for conjugation to proteins whose spectrophotometric absorptions, and whose metal chelate spectrophotometric absorptions, do not overlap with those of the proteins to which the chelating agents are chemically bonded.

Another problem with some prior art compositions is that the chelator must be activated by a reducing agent before forming the radionuclide chelate. If the protein conjugates are to be formed prior to formation of the radionuclide chelate, then the reducing agent employed for activating the complexing agent can degrade the protein. For example, the preferred chelating agents currently used for complexing technetium (Tc) and rhenium (Re) complex to the metals via sulfur-containing groups which must be reduced with a reducing agent (dithiothreitol) to activate the chelator before forming the radionuclide chelate. If the protein conjugate containing disulfide bonds is formed prior to reduction, then the reducing agent can degrade the protein. It would be highly desirable to have chelating agents capable of forming conjugates with proteins before complexing with radionuclides.

In summary, the various currently available radiolabeled antibodies and chelating agents employed for making immunoreactive conjugates by covalently bonding a chelating agent to the immunoreactive protein, and radionuclide complexes thereof for use in diagnostic imaging and targeted therapeutics suffer from one or more of the following disadvantages: 1) toxicity; 2) dispersion of une reagent due to rapid metabolism; 3) inadequate emission characteristics; 4) inefficient covalent bonding with protein for conjugate preparation; 5) slow complexation with metals; 6) unstable metal complexation, e.g., with respect to temperature, time or pH; 7) inability to form conjugates and remain stable in storage until metal complexation is desired; 8) inability to spectrophotometrically analyze the radionuclide complex reagent; and 9) inability to complex without activation steps that degrade protein.
In WO92/08494, targetting radioactive immunoreagents are described which are designed to address a number of the problems described above. The immunoreagents are based on pyridine or phenanthroline species.

### SUMMARY OF THE INVENTION

We have discovered complexes which solve the problems of the prior art discussed above. The complexes of this invention comprise a metal radionuclide ion, a complexing agent which is a derivative of a terpyridine, and optionally an immunoreactive group covalently bonded to the complexing agent through a protein reactive group
More particularly, in accordance with the invention, there is provided:

A complex of a complexing agent of formula 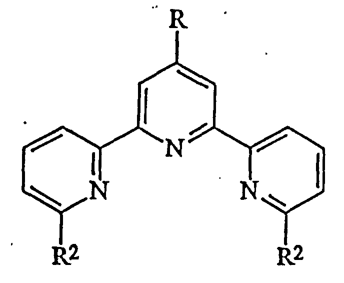 (where each R² represents a group CH₂N(CH₂COOH)₂ or a carboxyalkylthioalkyl group or together both R² groups represent a group of formula 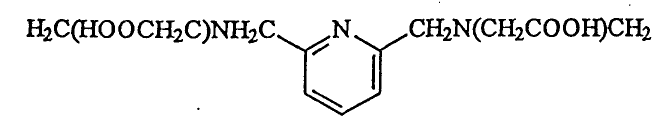 and R represents a protein reactive group optionally covalently bonded to an immuno-reactive organic compound; with the provisos that where each R² represents a group CH₂N(CH₂COOH)₂ then R is other than a p-methoxyphenyl group substituted in the 3-position by an amino, isothiocyanato or dimethylaminothiocarbonylamino group) and where both R² groups represent a group of formula 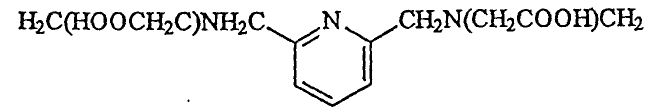 then R represents 3-amino-4-methoxyphenyl, or a salt thereof with a metal radionuclide ion.

This invention also provides therapeutic and diagnostic compositions comprising the above-described complexes.

This invention further provides use of an immunoreactive group containing complex as hereinbefore described in the manufacture of a medicament for use in diagnostic imaging.

It is an advantageous feature of this invention that the described complexes containing yttrium exhibit lower radiation toxicity, when compared to radioactive immunoreagents prepared with other yttrium chelators.

It is also an advantageous feature that the complexes of this invention are not rapidly metabolized and do not deleteriously disperse.

It is another advantageous feature that the described complexes efficiently form covalent bonds with proteins and other biological molecules.

Yet another advantageous feature of this invention is that the described complexes exhibit good emission characteristics and are readily subject to spectrophotometric analysis.

Additionally, protein conjugates of the complexing agents can be formed and stored until metal complexation is desired, and complexation can be accomplished without activation steps that degrade protein.

Moreover, the complexing agents rapidly complex with metals, and the resulting chelates exhibit excellent stability with respect to time, temperature and pH.

Other advantageous features of this invention will become readily apparent upon reference to the following description of the preferred embodiments.

### Description of Preferred Embodiments

The description which follows primarily concerns usage of the complexes in therapeutic and diagnostic imaging compositions and methods. In addition, the complexes are useful as diagnostic reagents, for example, radioimmunoelectrophoresis reagents.

The complexes of this invention comprise a metal radionuclide ion, a complexing agent, and optionally an immunoreactive group covalently bonded to the complexing agent through a protein reactive group as described above.

By "protein reactive group" is meant any group which can react with any functional groups typically found on or introduced into proteins. However, it is specifically contemplated that the protein reactive group can be conjugated to nonprotein biomolecules. Thus the protein reactive groups useful in the practice of this invention include those groups which can react with any biological molecule (including carbohydrates, nucleic acids and lipids) containing a reactive group, or a specific receptor-ligand interactive group, to form a linking group between the complexing agent and the immunoreactive group.

Preferred protein reactive groups can be selected from, but are not limited to: (1) A group that will react directly with the amine or sulfhydryl groups on the protein or biological molecule containing the immunoreactive group, for example, active halogen containing groups including, for example, chloromethylphenyl groups and chloroacetyl [Cl-CH₂CO-] groups, activated 2-leaving group substituted ethylsulfonyl and ethylcarbonyl groups such as 2-chloroethylsulfonyl and 2-chloroethylcarbonyl; vinylsulfonyl; vinylcarbonyl; epoxy; isocyanato; isothiocyanato; aldehyde; aziridine; succinimidoxycarbonyl; activated acyl groups such as carboxylic acid halides; mixed anhydrides and the like; and other groups known to be useful in conventional photographic gelatin hardening agents; (2) A group that can react readily with modified proteins or biological molecules containing the immunoreactive group, i.e., proteins or biological molecules containing the immunoreactive group modified to contain reactive groups such as those mentioned in (1) above, for example, by partial oxidation of the protein to introduce an aldehyde or a carboxylic acid, in which case the "protein reactive group" can be selected from amino, alkylamino, arylamino, hydrazino, alkylhydrazino, arylhydrazino, carbazido, semicarbazido, thiocarbazido, thiosemicarbazido, sulfhydryl, sulfhydrylalkyl, sulfhydrylaryl, hydroxy, carboxy, carboxyalkyl and carboxyaryl. The alkyl portions of the protein reactive group can contain from 1 to about 20 carbons atoms such as methyl, ethyl, propyl, isopropyl, butyl, s-butyl, t-butyl, 2-ethylhexyl, decyl, hexadecyl, octadecyl, etc. The aryl portions of the protein reactive group can contain from about 6 to about 20 carbon such as phenyl, naphthyl, phenanthryl, nitrophenyl, hydroxyphenyl, aminophenyl, hexadecylaminophenyl, octadecylaminophenyl, tolyl, xylyl, methoxyphenyl, 3-amino-4-methoxyphenyl, 4-methoxy-3-(N-methylhydrazinothioformamido)phenyl, 3-isocyanato-4-methoxyphenyl, 3-isothiocyanato-4-methoxyphenyl, methylthiophenyl, carboxyphenyl.

(3) A group that can be linked to the protein or biological molecule containing the immunoreactive group, or to the modified protein as noted in (1) and (2) above by use of a crosslinking agent. Certain useful crosslinking agents, such as, for example, difunctional gelatin hardeners, bisepoxides and bisisocyanates become a part of, i.e., a linking group in, the protein-complexing agent conjugate during the crosslinking reaction. Other useful crosslinking agents, however, facilitate the crosslinking, for example, as consumable catalysts, and are not present in the final conjugate. Examples of such crosslinking agents are carbodiimide and carbamoylonium crosslinking agents as disclosed in U.S. Patent 4,421,847, the disclosure of which is hereby incorporated herein by reference in its entirety, and the dication ethers of U.S. Patent 4,877,724, the disclosure of which is hereby incorporated herein by reference in its entirety. With these crosslinking agents, one of the reactants must have a carboxyl group and the other an amine or sulfhydryl group. The crosslinking agent first reacts selectively with the carboxyl group, then is split out during reaction of the "activated" carboxyl group with an amine to form an amide linkage between the protein and the metal complexing agent, thus covalently bonding the two moieties. An advantage of this approach is that crosslinking of like molecules, e.g., complexing agents with complexing agents, is avoided, whereas the reaction of difunctional crosslinking agents is nonselective so that unwanted crosslinked molecules are obtained. Especially preferred protein reactive groups include amino and isothiocyanato.

The polypyridine complexing agents having metal complexing sites, e.g., heteroatoms and iminodiacetate groups can be prepared by techniques known in the art. Suitable reaction schemes are illustrated in U.S. Patent 4,837,169 and U.S. Patent 4,859,777, the disclosures of which are hereby incorporated herein by reference.

For a full descripton on how to prepare the complexes of the invention, attention is drawn to WO 92/08494 and WO 93/21957.

The complexes of this invention includes a radionuclide ion. The radionuclide ion can be selected, for example, from Sc, Fe, Pb, Ga, Y, Bi, Mn, Cu, Cr, Zn, Ge, Mo, Tc, Ru, In, Sn, Sm, Sb, W, Re, Po, Ta and Tl ions. Preferred radionuclides include ⁴⁴Sc⁺⁺⁺, ^{64,67}Cu⁺⁺, ¹¹¹In⁺⁺⁺, ²¹²P_{b}⁺⁺, ⁶⁸Ga⁺⁺, ⁹⁰Y⁺⁺⁺ and ²¹²Bi⁺⁺⁺ ions. Of these, the most preferred are ⁹⁰Y⁺⁺⁺ ions.

The metal radionuclide ion and the complexing agent are easily complexed by merely mixing an aqueous solution of the complexing agent with a metal radionuclide salt in an aqueous solution preferably having a pH of 4 to 11. The salt can be any water soluble salt of the metal such as halogen salts. The chelate is generally prepared in aqueous solution at a pH of between 5 and 9 and preferably from 6 to 8. The complex optionally is mixed with buffers such as acetate, phosphate and borate to produce the optimum pH.

As used herein the term "immunoreactive group" is meant to include any organic compound which is capable of covalently bonding to the complexing agent and which is found in a living organism or is useful in the diagnosis, treatment or genetic engineering of cellular material or living organisms, and which has a capacity for interaction with another component which may be found in biological fluids or associated with cells to be treated such as tumor cells.

Depending upon the intended use, the immunoreactive group can be selected from a wide variety of naturally occurring or synthetically prepared materials, including, but not limited to enzymes, amino acids, peptides, polypeptides, proteins, lipoproteins, glycoproteins, hormones, drugs (for example digoxin, phenytoin, phenobarbitol, thyrozine, triiodothyronine, gentamicin, carbamazepine, and theophylline), steroids, vitamins, polysaccharides, viruses, protozoa, fungi, parasites, rickettsia, molds, and components thereof, blood components, tissue and organ components, pharmaceuticals, haptens, lectins, toxins, nucleic acids (including oligonucleotides), antibodies (including genetically engineered antibodies and fragments thereof), antibody fragments, antigenic materials (including proteins and carbohydrates), avidin and derivatives thereof, biotin and derivatives thereof, and others known to one skilled in the art.

Preferred immunoreactive groups for use in the practice of this invention are those which have a receptor molecule specific to a ligand of interest. Thus, a specific binding reaction involving the reagent can be used for the targeting expected. Examples of such ligand-receptor complexes include, but are not limited to antibody-antigen, avidin-biotin, repressor (inducer) - promoter of operons and sugar-lectin complexes. Additionally, complementary nucleic acids, i.e., a hybridized product of complementary strands, are also considered specific binding materials as the term is used herein.

Particularly preferred immunoreactive groups include (1) any substance which, when presented to an immunocompetent host, will result in the production of a specific antibody capable of binding with that substance, or (2) the antibody so produced, which participates in an antigen-antibody reaction. Thus, the immunoreactive group can be an antigenic material, an antibody, or an anti-antibody. Both monoclonal and polyclonal antibodies are useful. The antibodies can be whole molecules or various fragments thereof, as long as they contain at least one reactive site for reaction with the reactive groups on the complexing agent or with linking groups as described herein. Specifically contemplated as being included within these preferred immunoreactive groups are antibodies and proteins produced by the techniques of molecular biology. Such techniques are well known in the art and are described, for example, in U.S. Patent Nos. 4,816,397 and 4,816,567.

In certain embodiments, the immunoreactive group can be an enzyme which has a reactive group for attachment to the complexing agent. Representative enzymes include, but are not limited to, aspartate aminotransaminase, alanine aminotransaminase, lactate dehydrogenase, creatine phosphokinase, dihydrofolate reductase, gamma glutamyl transferase, alkaline acid phosphatase, prostatic acid phosphatase, horseradish peroxidase and various esterases.

If desired, the immunoreactive group can be modified or chemically altered to provide reactive groups for attaching to the complexing agent by techniques known to those skilled in the art. Such techniques include the'use of linking moieties and chemical modification such as described in WO-A-89/02931 and WO-A-89/02932, which are directed to modification of oligonucleotides, and U.S. Patent 4,719,182 the disclosures of all of which are hereby incorporated herein by reference in their entirety.

Two highly preferred uses of the complexes of this invention are for the diagnostic imaging of tumors and the radiological treatment of tumors. Preferred immunological groups therefore include antibodies to tumor-associated antigens. Specific examples include B72.3 antibodies (described in U.S. Patents Nos. 4,522,918 and 4,612,282) which recognize colorectal tumors, 9.2.27 anti-melanoma antibodies, D612 antibodies which recognize colorectal tumors, UJ13A antibodies which recognize small cell lung carcinomas, NRLU-10 (Tfs-2) antibodies which recognize small cell lung carcinomas and colorectal tumors, 7E11C5 antibodies which recognize prostate tumors, CC49 antibodies which recognize colorectal tumors, TNT antibodies which recognize necrotic tissue, PR1A3 antibodies, which recognize colon carcinoma [Richman, P.I. and Bodmer, W.F. (1987) Int., J, Cancer Vol. 39, pp. 317-328], ING-1 and other genetically engineered antibodies, which are described in International Patent Publication WO-A-90/02569, B174 antibodies (developed at Biomira, Inc. of Edmonton, Canada, which recognize squamous cell carcinomas, B43 antibodies which are reactive with certain lymphomas and leukemias and others which may be of particular interest.

Such antibodies and other useful immunological groups described above are large, complex molecules having multiple sites for appendage of the complexing agent. Consequently, the immunoreactive group can have appended to it additional complexing agents via one of the protein reactive groups. Thus, the term immunoreactive group is intended to include immunological groups having complexing agent molecules bonded thereto through one or more protein reactive groups.

Additionally, an antibody or fragment thereof containing a carbohydrate region can be attached to the complexing agent through the carbohydrate region of the antibody, such as described in U.S. Patent 4,937,183, the disclosure of which is hereby incorporated herein by reference in its entirety. Useful methods for attaching an antibody are also described in U.S. Patents Nos. 4,671,958; 4,699,784; 4,741,900; and 4,867,973. The term "protein reactive group" as defined herein is intended to include such linkages.

Other techniques for performing the covalent binding of the immunoreactive group to the radioactive metal complexing agents are known in the art and include simply mining the materials together.

The complexes of this invention can contain any ratio of metal radionuclide ion to complexing agent. In preferred embodiments, the mole ratio of metal ion to complexing agent is from about 1:100 to about 1:1.

The ratio of the complexing agent to the immunoreactive group can vary widely from about 0.5:1 to 10:1 or more. In some embodiments, the mole ratio of complexing agent to immunoreactive groups is from about 1:1 to about 6:1.

The following examples further illustrate the invention:

### Preparation 1 Macrocyclic Chelator: Disodium 1.7-dicarboxymethyl-[4'-(3-amino-4-methoxyphenyl)-2,2':6'2"-terpyridinol-1,4,7,10,13,16-hexaaza-18-crown-6

### Part A - 6,6"-Dicyano-4'-(4-methoxyphenyl)-2,2':6',2"-terpyridine

A mixture of 6,6"-dibromo-4'-(4-methoxyphenyl)-2,2':6',2"-terpyridine (0.0745 mol), cupurous cyanide (0.296 mol), sodium cyanide (0.297 mol), and 300 mL of dimethylformamide is allowed to react at 160°C for 6 h. After cooling, the reaction mixture was transferred into 1500 mL of water and the resulting mixture is stirred for 2 h and filtered. The solid residue is dissolved in a solution of sodium cyanide (200 g in 400 mL water) and the resulting solution is stirred overnight. The desired solid product is filtered, triturated with water (6x600 mL), and dried to yield a white powder.

### Part B. 6,6"-Bis(aminomethyl)-4'-(4-methoxyphenyl)-2,2':6'2"-terpyridine

A mixture of 6,6"-dicyano-4'-(4-methoxyphenyl)-2,2':6',2"-terpyridine ( 4 mmol) and 480 mg of 10% Pd/C in 130 mL of acetic acid is hydrogenated (H₂, 50 psi) at 45°C for 22 h. The reaction mixture is filtered and the solvent is stripped to yield the desired diamine as the acetate salt, which is triturated with methanol followed by removal of solvent and drying to yield the desired diamine acetate.

### Part C. 6,6"-Bis(aminomethyl)-4'-(4-methoxyphenyl)-2,2':6'2"-terpyridine pyridine-2,6-dicarboxylic acid cyclic dilactam

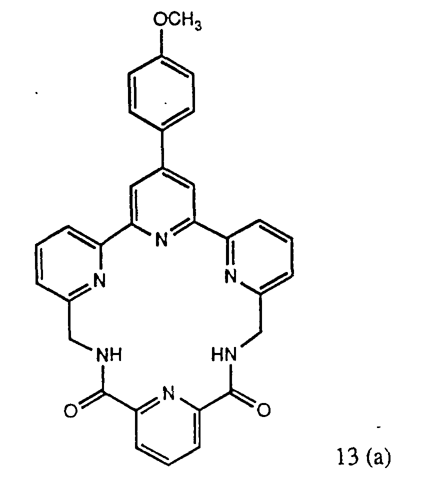

A solution of 6,6"-bis(aminomethyl)-4'-(4-methoxyphenyl)-2,2':6'2"-terpyridine (2 mmol) in tetrahydrofuran (41 mL) and a solution of diisopropylamine (4 mmol) in dimethylformamide (9 mL) are added with stirring into 350 mL of THF. To the above solution is added dropwise a solution of 2,6-pyridine-dicarbonyl chloride in THF (50 mL) and the reaction mixture is stirred overnight. The solvents are distilled under diminished pressure and the residue dissolved in 250 mL of CH₂Cl₂. The organic layer is washed successively with water (2x30 mL), 0.01 N HCl solution (2x25 mL), water (1x20 mL), 5% NaHCO₃ solution (2x25 mL), and water (2x20 mL). After drying ( Na₂SO₄), the solvent is distilled to yield the desired dilactam which is then purified by silica gel chromatography.

### Part D. Disodium 1,7-dicarboxymethyl-[4'-(3-amino-4-methoxyphenyl)-2,2':6'2"-terpyridino]-1,4,7,10,13,16-hexaaza-18-crown-6

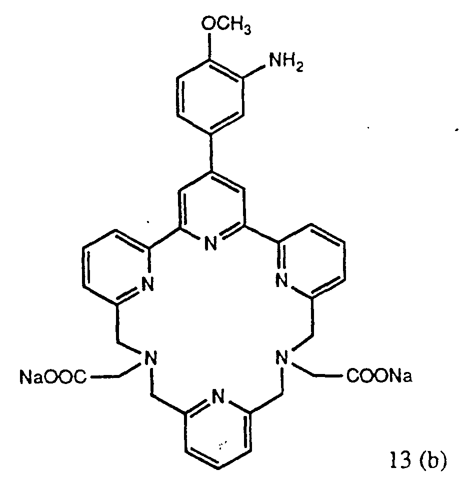

The cyclic dilactam (a) of Part C above is reduced with diborane to provide the diamine which is reacted with ethyl bromoacetate to provide the bis(ethyl acetate). This is then nitrated with nitric acid and sulfuric acid to provide the 4'-(4-methoxy-3-nitrophenyl)-2,2':6'2"-terpyridinyl derivative. The nitro group is then reduced with ammonium formate in the presence of palladium on carbon, and the ester groups are saponified with sodium hydroxide to provide the desired macrocylic chelate (b).

## Claims (Claims for the following Contracting State(s): AT, BE, CH, LI, DE, DK, FR, GB, IT, LU MC, NL, SE)

1. A complex of a complexing agent of formula 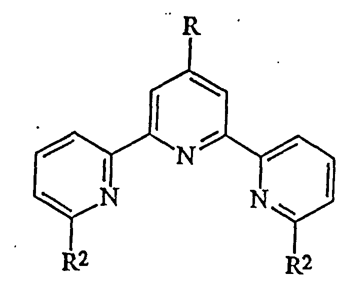 (where each R² represents a group CH₂N(CH₂COOH)₂ or a carboxyalkylthioalkyl group or together both R² groups represent a group of formula 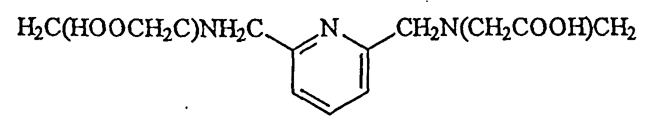 and R represents a protein reactive group optionally covalently bonded to an immuno-reactive organic compound; with the provisos that where each R² represents a group CH₂N(CH₂COOH)₂ then R is other than a p-methoxyphenyl group substituted in the 3-position by an amino, isothiocyanato or dimethylaminothiocarbonylamino group) and where both R² groups represent a group of formula 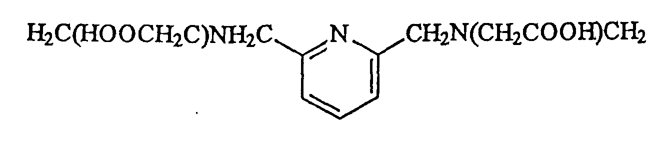 then R represents 3-amino-4-methoxyphenyl, or a salt thereof with a metal radionuclide ion.

2. A complex as claimed in claim 1 wherein group R comprises a reactive group selected from amino, alkylamino, arylamino, hydrazino, alkylhydrazino, arylhydrazino, carbazido, semicarbazido, thiocarbazido, thiosemicarbazido, sulfhydryl, sulfhydrylalkyl, sulfhydrylaryl, hydroxy, carboxy, carboxyalkyl, carboxyaryl, active halogen containing groups, 2-leaving group-substituted ethylcarbonyl, vinylsulfonyl, vinylcarbonyl, epoxy, isocyanato, isothiocyanato, aldehyde, aziridine, succinimidoxycarbonyl activated acyl groups, and mixed anhydrides, optionally covalently bonded to an immuno-reactive organic material.

3. A complex as claimed in either of claims 1 and 2 wherein group R comprises an immuno-reactive group selected from enzymes, amino acids, polypeptides, proteins, lipoproteins, glycoproteins, hormones, drugs, steroids, vitamins, polysaccharides, pharmaceuticals, haptens, lectins, toxins, nucleic acids, antibodies, antigenic materials, avidin and biotin.

4. A complex as claimed in claim 3 wherein group R comprises an antibody.

5. A complex as claimed in claim 4 wherein group R comprises an antibody selected from B72.3, 9.2.27, D612, UJ13A, NRLU-10, 7E11C5, CC49, TNT, PRIA3, ING-1, B174 and B43.

6. A complex as claimed in claim 5 wherein group R comprises the antibody ING-1.

7. A complex as claimed in any one of claims 1 to 6 wherein said metal radionuclide ion is ⁹⁰Y³⁺.

8. A diagnostic or therapeutic composition comprising an immuno-reactive group containing complex as claimed in any one of claims 1 to 7, together with a pharmaceutically acceptable carrier.

9. The use of an immunoreactive group containing complex as claimed in any one of claims 1 to 7 in the manufacture of a medicament for use in diagnostic imaging.

## Claims (Claims for the following Contracting State(s): ES, GR)

1. A complex of a complexing agent of formula 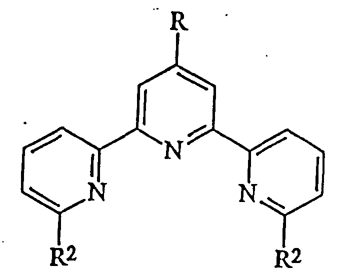 (where each R² represents a group CH₂N(CH₂COOH)₂ or a carboxyalkylthioalkyl group or together both R² groups represent a group of formula 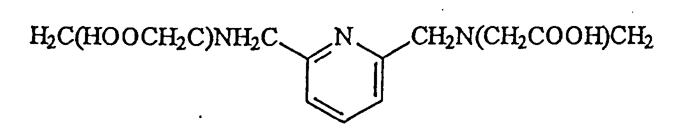 and R represents a protein reactive group optionally covalently bonded to an immuno-reactive organic compound; with the provisos that where each R² represents a group CH₂N(CH₂COOH)₂ then R is other than a p-methoxyphenyl group substituted in the 3-position by an amino, isothiocyanato or dimethylaminothiocarbonylamino group) and where both R² groups represent a group of formula 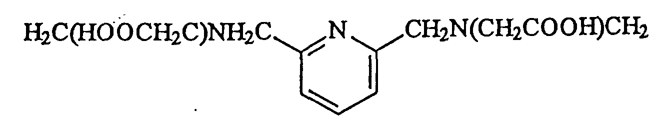 then R represents 3-amino-4-methoxyphenyl, or a salt thereof with a metal radionuclide ion.

2. A complex as claimed in claim 1 wherein group R comprises a reactive group selected from amino, alkylamino, arylamino, hydrazino, alkylhydrazino, arylhydrazino, carbazido, semicarbazido, thiocarbazido, thiosemicarbazido, sulfhydryl, sulfhydrylalkyl, sulfhydrylaryl, hydroxy, carboxy, carboxyalkyl, carboxyaryl, active halogen containing groups, 2-leaving group-substituted ethylcarbonyl, vinylsulfonyl, vinylcarbonyl, epoxy, isocyanato, isothiocyanato, aldehyde, aziridine, succinimidoxycarbonyl activated acyl groups, and mixed anhydrides, optionally covalently bonded to an immuno-reactive organic material.

3. A complex as claimed in either of claims 1 and 2 wherein group R comprises an immuno-reactive group selected from enzymes, amino acids, polypeptides, proteins, lipoproteins, glycoproteins, hormones, drugs, steroids, vitamins, polysaccharides, pharmaceuticals, haptens, lectins, toxins, nucleic acids, antibodies, antigenic materials, avidin and biotin.

4. A complex as claimed in claim 3 wherein group R comprises an antibody.

5. A complex as claimed in claim 4 wherein group R comprises an antibody selected from B72.3, 9.2.27, D612, UJ13A, NRLU-10, 7E11C5, CC49, TNT, PRIA3, ING-1, B174 and B43.

6. A complex as claimed in claim 5 wherein group R comprises the antibody ING-1.

7. A complex as claimed in any one of claims 1 to 6 wherein said metal radionuclide ion is ⁹⁰Y³⁺.

8. A diagnostic or therapeutic composition comprising an immuno-reactive group containing complex as claimed in any one of claims 1 to 7, together with a pharmaceutically acceptable carrier.

9. The use of an immunoreactive group containing complex as claimed in any one of claims 1 to 7 in the manufacture of a medicament for use in diagnostic imaging.

10. A process for the preparation of a complex of a complexing agent of formula 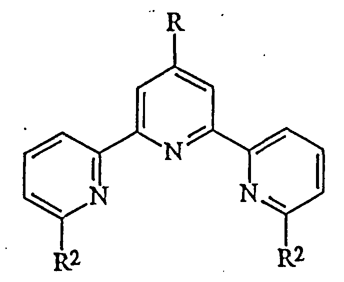 (where each R² represents a group CH₂N(CH₂COOH)₂ or a carboxyalkylthioalkyl group or together both R² groups represent a group of formula 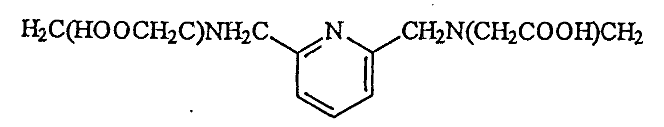 and R represents a protein reactive group optionally covalently bonded to an immuno-reactive organic compound; with the provisos that where each R² represents a group CH₂N(CH₂COOH)₂ then R is other than a p-methoxyphenyl group substituted in the 3-position by an amino, isothiocyanato or dimethylaminothiocarbonylamino group) and where both R² groups represent a group of formula 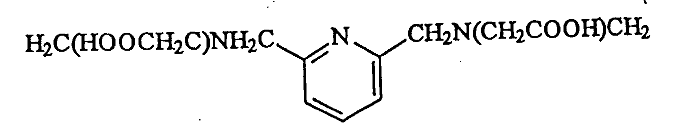 then R represents 3-amino-4-methoxyphenyl, or a salt thereof with a metal radionuclide ion comprising reacting said complexing agent with said metal radionuclide ion.

## Patentansprüche (Patentansprüche für folgende(n) Vertragsstaat(en): AT, BE, CH, LI, DE, DK, FR, GB, IT, LU, MC, NL, SE)

1. Komplex aus einem Komplexierungsmittel der Formel 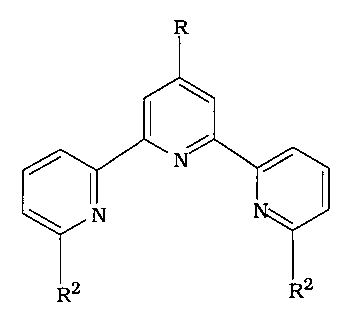 (worin jedes R² eine Gruppe CH₂N(CH₂COOH)₂ oder eine Carboxyalkylthioalkylgruppe bedeutet oder zusammen beide R²-Gruppen eine Gruppe der Formel 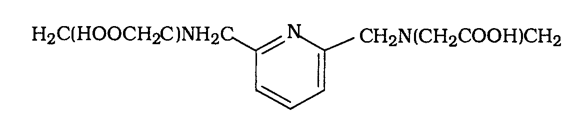 bedeuten und R eine Protein-reaktive Gruppe bedeutet, welche wahlweise kovalent an eine immonureaktive organische Verbindung gebunden ist; mit den Maßgaben, dass, wenn jedes R² eine Gruppe CH₂N(CH₂COOH)₂ bedeutet, dann R verschieden ist von einer p-Methoxyphenylgruppe, substituiert in der 3-Position durch eine Amino-, Isothiocyanato- oder Dimethylaminothiocarbonylaminogruppe), und wenn beide R²-Gruppen eine Gruppe der Formel 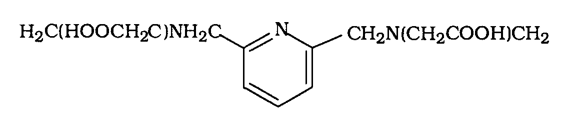 bedeuten, dann R die Bedeutung 3-Amino-4-methoxyphenyl hat, oder ein Salz hiervon, mit einem Metallradionuclidion.

2. Komplex nach Anspruch 1, wobei die Gruppe R eine reaktive Gruppe umfaßt, gewählt aus Amino, Alkylamino, Arylamino, Hydrazino, Alkylhydrazino, Arylhydrazino, Carbazido, Semicarbazido, Thiocarbazido, Thiosemicarbazido, Sulfhydryl, Sulfhydrylalkyl, Sulfhydrylaryl, Hydroxy, Carboxy, Carboxyalkyl, Carboxyaryl, aktives Halogen enthaltenden Gruppen, 2-Abspaltguppen-substituiertes Ethylcarbonyl, Vinylsulfonyl, Vinylcarbonyl, Epoxy, Isocyanato, Isothiocyanato, Aldehyd, Aziridin, Succinimidoxycarbonyl-aktivierte Acylgruppen und gemischte Anhydride, wahlweise kovalent gebunden an ein immunoreaktives organisches Material.

3. Komplex nach Anspruch 1 und/oder 2, wobei die Gruppe R eine immunoreaktive Gruppe umfaßt, gewählt aus Enzymen, Aminosäuren, Polypeptiden, Proteinen, Lipoproteinen, Glycoproteinen, Hormonen, Drogen, Steroiden, Vitaminen, Polysacchariden, Pharmazeutika, Haptenen, Lectinen, Toxinen, Nucleinsäuren, Antikörpern, antigenen Materialien, Avidin und Biotin.

4. Komplex nach Anspruch 3, wobei die Gruppe R einen Antikörper umfaßt.

5. Komplex nach Anspruch 4, wobei die Gruppe R einen Antikörper umfaßt, gewählt aus B72.3, 9.2.27, D612, UJ13A, NRLU-10, 7E11C5, CC49, TNT, PRIA3, ING-1, B174 und B43.

6. Komplex nach Anspruch 5, wobei die Gruppe R den Antikörper ING-1 umfaßt.

7. Komplex nach mindestens einem der Ansprüche 1 bis 6, wobei das Metallradionuclidion ⁹⁰Y³⁺ ist.

8. Diagnostische oder therapeutische Zusammensetzung, umfassend einen eine immunoreaktive Gruppe enthaltenden Komplex nach mindestens einem der Ansprüche 1 bis 7, zusammen mit einem pharmazeutisch annehmbaren Träger.

9. Verwendung eines eine immunoreaktive Gruppe enthaltenden Komplexes nach mindestens einem der Ansprüche 1 bis 7 zur Herstellung eines Arzneimittels zur Verwendung bei diagnostischen Bilderzeugungen.

## Patentansprüche (Patentansprüche für folgende(n) Vertragsstaat(en): ES, GR)

1. Komplex aus einem Komplexierungsmittel der Formel 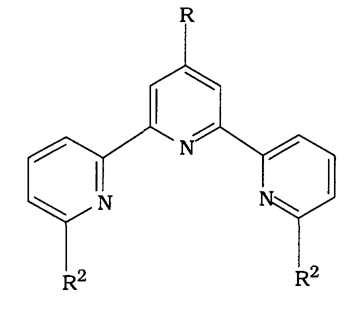 (worin jedes R² eine Gruppe CH₂N(CH₂COOH)₂ oder eine Carboxyalkylthioalkylgruppe bedeutet oder zusammen beide R²-Gruppen eine Gruppe der Formel 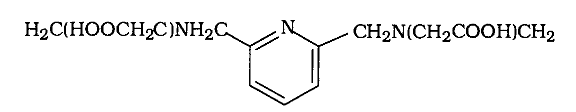 bedeuten und R eine Protein-reaktive Gruppe bedeutet, welche wahlweise kovalent an eine immonureaktive organische Verbindung gebunden ist; mit den Maßgaben, dass, wenn jedes R² eine Gruppe CH₂N(CH₂COOH)₂ bedeutet, dann R verschieden ist von einer p-Methoxyphenylgruppe, substituiert in der 3-Position durch eine Amino-, Isothiocyanato- oder Dimethylaminothiocarbonylaminogruppe), und wenn beide R²-Gruppen eine Gruppe der Formel 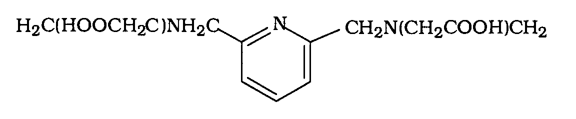 bedeuten, dann R die Bedeutung 3-Amino-4-methoxyphenyl hat, oder ein Salz hiervon, mit einem Metallradionuclidion.

2. Komplex nach Anspruch 1, wobei die Gruppe R eine reaktive Gruppe umfaßt, gewählt aus Amino, Alkylamino, Arylamino, Hydrazino, Alkylhydrazino, Arylhydrazino, Carbazido, Semicarbazido, Thiocarbazido, Thiosemicarbazido, Sulfhydryl, Sulfhydrylalkyl, Sulfhydrylaryl, Hydroxy, Carboxy, Carboxyalkyl, Carboxyaryl, aktives Halogen enthaltenden Gruppen, 2-Abspaltguppen-substituiertes Ethylcarbonyl, Vinylsulfonyl, Vinylcarbonyl, Epoxy, Isocyanato, Isothiocyanato, Aldehyd, Aziridin, Succinimidoxycarbonyl-aktivierte Acylgruppen und gemischte Anhydride, wahlweise kovalent gebunden an ein immunoreaktives organisches Material.

3. Komplex nach Anspruch 1 und/oder 2, wobei die Gruppe R eine immunoreaktive Gruppe umfaßt, gewählt aus Enzymen, Aminosäuren, Polypeptiden, Proteinen, Lipoproteinen, Glycoproteinen, Hormonen, Drogen, Steroiden, Vitaminen, Polysacchariden, Pharmazeutika, Haptenen, Lectinen, Toxinen, Nucleinsäuren, Antikörpern, antigenen Materialien, Avidin und Biotin.

4. Komplex nach Anspruch 3, wobei die Gruppe R einen Antikörper umfaßt.

5. Komplex nach Anspruch 4, wobei die Gruppe R einen Antikörper umfaßt, gewählt aus B72.3, 9.2.27, D612, UJ13A, NRLU-10, 7E11C5, CC49, TNT, PRIA3, ING-1, B174 und B43.

6. Komplex nach Anspruch 5, wobei die Gruppe R den Antikörper ING-1 umfaßt.

7. Komplex nach mindestens einem der Ansprüche 1 bis 6, wobei das Metallradionuclidion ⁹⁰Y³⁺ ist.

8. Diagnostische oder therapeutische Zusammensetzung, umfassend einen eine immunoreaktive Gruppe enthaltenden Komplex nach mindestens einem der Ansprüche 1 bis 7, zusammen mit einem pharmazeutisch annehmbaren Träger.

9. Verwendung eines eine immunoreaktive Gruppe enthaltenden Komplexes nach mindestens einem der Ansprüche 1 bis 7 zur Herstellung eines Arzneimittels zur Verwendung bei diagnostischen Bilderzeugungen.

10. Verfahren zur Herstellung eines Komplexes aus einem Komplexierungsmittel der Formel 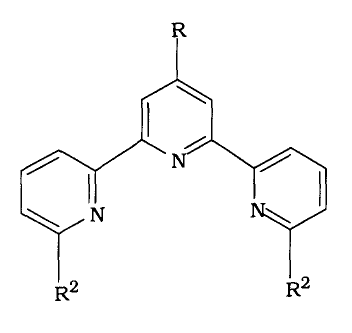 (worin jedes R² eine Gruppe CH₂N(CH₂COOH)₂ oder eine Carboxyalkylthioalkylgruppe bedeutet oder zusammen beide R²-Gruppen eine Gruppe der Formel 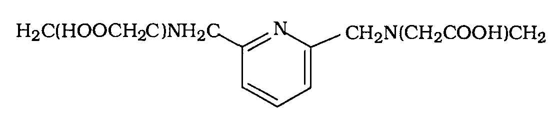 bedeuten und R eine Protein-reaktive Gruppe bedeutet, welche wahlweise kovalent an eine immonureaktive organische Verbindung gebunden ist; mit den Maßgaben, dass, wenn jedes R² eine Gruppe CH₂N(CH₂COOH)₂ bedeutet, dann R verschieden ist von einer p-Methoxyphenylgruppe, substituiert in der 3-Position durch eine Amino-, Isothiocyanato- oder Dimethylaminothiocarbonylaminogruppe), und wenn beide R²-Gruppen eine Gruppe der Formel 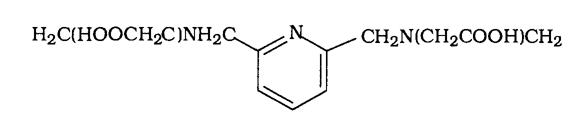 bedeuten, dann R die Bedeutung 3-Amino-4-methoxyphenyl hat, oder ein Salz hiervon, mit einem Metallradionuclidion, umfassend das Umsetzen des Komplexierungsmittels mit dem Metallradionuclidion.

## Revendications (Revendications pour l'(les) Etat(s) contractant(s) suivant(s): AT, BE, CH, LI, DE, DK, FR, GB, IT, LU, MC, NL, SE)

1. Complexe d'un agent complexant de formule 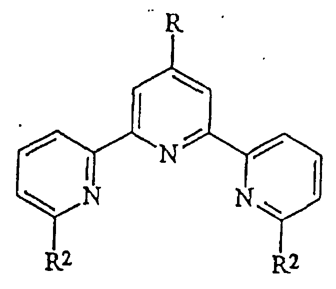 (dans laquelle chaque R² représente un groupe CH₂N(CH₂COOH)₂ ou un groupe carboxyalkylthioalkyle ou bien, ensemble, les deux groupes R² représentent un groupe de formule 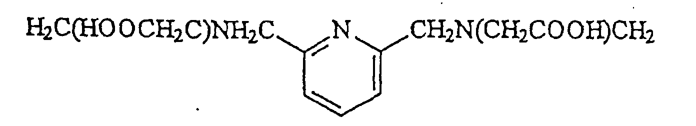 et R représente un groupe réactif avec une protéine, éventuellement lié de façon covalente à un composé organique immuno-réactif ; étant entendu que lorsque chaque R² représente un groupe CH₂N(CH₂COOH)₂ alors R est autre qu'un groupe p-méthoxyphényle substitué en position 3 par un groupe amino, isothiocyanato ou diméthylaminothiocarbonylamino) et lorsque les deux groupes R² représentent un groupe de formule 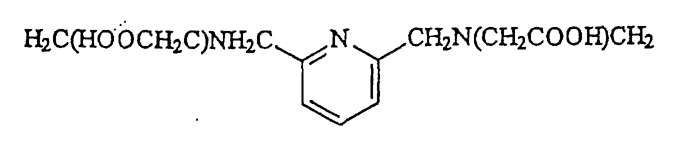 alors R représente un groupe 3-amino-4-méthoxyphényle, ou un sel de celui-ci avec un ion de radionucléide métallique.

2. Complexe selon la revendication 1, dans lequel le groupe R comprend un groupe réactif choisi parmi un groupe amino, alkylamino, arylamino, hydrazino, alkylhydrazino, arylhydrazino, carbazido, semicarbazido, thiocarbazido, thiosemicarbazido, sulfhydryle, sulfhydrylalkyle, sulfhydrylaryle, hydroxy, carboxy, carboxyalkyle, carboxyaryle, des groupes contenant de l'halogène actif, un groupe éthylcarbonyle substitué par un groupe partant en 2, vinylsulfonyle, vinylcarbonyle, époxy, isocyanato, isothiocyanato, aldéhyde, aziridine, des groupes acyles activés par le succinimidoxycarbonyle, et des anhydrides mixtes, éventuellement lié de façon covalente à un matériau organique immuno-réactif.

3. Complexe selon l'une quelconque des revendications 1 et 2, dans lequel le groupe R comprend un groupe immuno-réactif choisi parmi des enzymes, des acides aminés, des polypeptides, des protéines, des lipoprotéines, des glycoprotéines, des hormones, des médicaments, des stéroïdes, des vitamines, des polysaccharides, des produits pharmaceutiques, des haptènes, des lectines, des toxines, des acides nucléiques, des anticorps, des matériaux antigéniques, l'avidine et la biotine.

4. Complexe selon la revendication 3, dans lequel le groupe R comprend un anticorps.

5. Complexe selon la revendication 4, dans lequel le groupe R comprend un anticorps choisi parmi B72.3, 9.2.27, D612, UJ13A, NRLU-10, 7E11C5, CC49, TNT, PRIA3, ING-1, B174 et B43.

6. Complexe selon la revendication 5, dans lequel le groupe R comprend l'anticorps ING-1.

7. Complexe selon l'une quelconque des revendications 1 à 6, dans lequel ledit ion de radionucléide métallique est ⁹⁰Y³⁺.

8. Composition diagnostique ou thérapeutique comprenant un complexe contenant un groupe immuno-réactif tel que revendiqué dans l'une quelconque des revendications 1 à 7, avec un véhicule pharmaceutiquement acceptable.

9. Utilisation d'un complexe contenant un groupe immuno-réactif tel que revendiqué dans l'une quelconque des revendications 1 à 7, dans la fabrication d'un médicament pour utilisation en imagerie diagnostique.

## Revendications (Revendications pour l'(les) Etat(s) contractant(s) suivant(s): ES, GR)

1. Complexe d'un agent complexant de formule 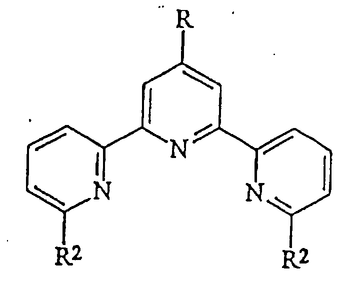 (dans laquelle chaque R² représente un groupe CH₂N(CH₂COOH)₂ ou un groupe carboxyalkylthioalkyle ou bien, ensemble, les deux groupes R² représentent un groupe de formule 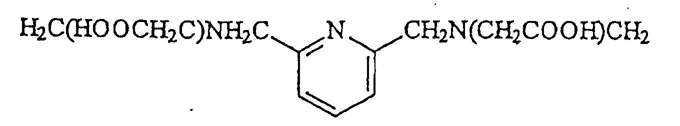 et R représente un groupe réactif avec une protéine, éventuellement lié de façon covalente à un composé organique immuno-réactif ; étant entendu que lorsque chaque R² représente un groupe CH₂N(CH₂COOH)₂ alors R est autre qu'un groupe p-méthoxyphényle substitué en position 3 par un groupe amino, isothiocyanato ou diméthylaminothiocarbonylamino) et lorsque les deux groupes R² représentent un groupe de formule 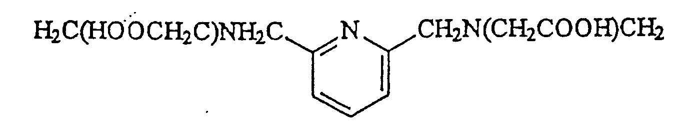 alors R représente un groupe 3-amino-4-méthoxyphényle, ou un sel de celui-ci avec un ion de radionucléide métallique.

2. Complexe selon la revendication 1, dans lequel le groupe R comprend un groupe réactif choisi parmi un groupe amino, alkylamino, arylamino, hydrazino, alkylhydrazino, arylhydrazino, carbazido, semicarbazido, thiocarbazido, thiosemicarbazido, sulfhydryle, sulfhydrylalkyle, sulfhydrylaryle, hydroxy, carboxy, carboxyalkyle, carboxyaryle, des groupes contenant de l'halogène actif, un groupe éthylcarbonyle substitué par un groupe partant en 2, vinylsulfonyle, vinylcarbonyle, époxy, isocyanato, isothiocyanato, aldéhyde, aziridine, des groupes acyles activés par le succinimidoxycarbonyle, et des anhydrides mixtes, éventuellement lié de façon covalente à un matériau organique immuno-réactif.

3. Complexe selon l'une quelconque des revendications 1 et 2, dans lequel le groupe R comprend un groupe immuno-réactif choisi parmi des enzymes, des acides aminés, des polypeptides, des protéines, des lipoprotéines, des glycoprotéines, des hormones, des médicaments, des stéroïdes, des vitamines, des polysaccharides, des produits pharmaceutiques, des haptènes, des lectines, des toxines, des acides nucléiques, des anticorps, des matériaux antigéniques, l'avidine et la biotine.

4. Complexe selon la revendication 3, dans lequel le groupe R comprend un anticorps.

5. Complexe selon la revendication 4, dans lequel le groupe R comprend un anticorps choisi parmi B72.3, 9.2.27, D612, UJ13A, NRLU-10, 7E11C5, CC49, TNT, PRIA3, ING-1, B174 et B43.

6. Complexe selon la revendication 5, dans lequel le groupe R comprend l'anticorps ING-1.

7. Complexe selon l'une quelconque des revendications 1 à 6, dans lequel ledit ion de radionucléide métallique est ⁹⁰Y³⁺.

8. Composition diagnostique ou thérapeutique comprenant un complexe contenant un groupe immuno-réactif tel que revendiqué dans l'une quelconque des revendications 1 à 7, avec un véhicule pharmaceutiquement acceptable.

9. Utilisation d'un complexe contenant un groupe immuno-réactif tel que revendiqué dans l'une quelconque des revendications 1 à 7, dans la fabrication d'un médicament pour utilisation en imagerie diagnostique.

10. Procédé pour la préparation d'un complexe d'un agent complexant de formule 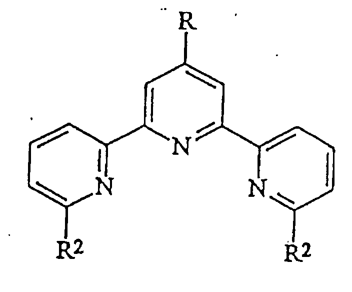 (dans laquelle chaque R² représente un groupe CH₂N(CH₂COOH)₂ ou un groupe carboxyalkylthioalkyle ou bien, ensemble, les deux groupes R² représentent un groupe de formule 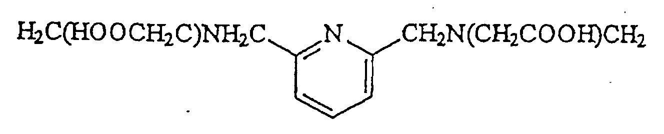 et R représente un groupe réactif avec une protéine, éventuellement lié de façon covalente à un composé organique immuno-réactif ; étant entendu que lorsque chaque R² représente un groupe CH₂N(CH₂COOH)₂ alors R est autre qu'un groupe p-méthoxyphényle substitué en position 3 par un groupe amino, isothiocyanato ou diméthylaminothiocarbonylamino) et lorsque les deux groupes R² représentent un groupe de formule 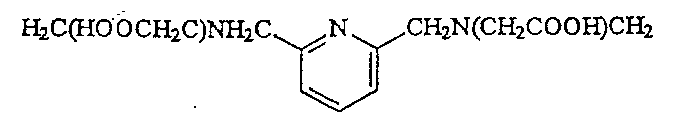 alors R représente un groupe 3-amino-4-méthoxyphényle, ou d'un sel de celui-ci avec un ion de radionucléide métallique, comprenant la réaction dudit agent complexant avec ledit ion de radionucléide métallique.
